# EUROPÄISCHE PATENTSCHRIFT

(11) **EP 0 387 325 B1**
(45) Veröffentlichungstag und Bekanntmachung des Hinweises auf die Patenterteilung: **08.02.1995**
(21) Anmeldenummer: 89909564.0
(22) Anmeldetag: 01.09.1989
(51) Int. Cl.: C07J 9/00, A61K 31/575, C07J 41/00, A61K 47/28, A61K 7/48

(54) **2-HYDROXY-N,N,N-TRIMETHYL-ETHANAMINIUMSALZE VON 5$g(b)-CHOLAN-24-SÄURE-DERIVATEN**
2-HYDROXY-N,N,N-TRIMETHYLETHANAMINIUM SALTS OF 5$g(b)-CHOLANE-24-ACID DERIVATES
SELS 2-HYDROXY-N,N,N,-TRIMETHYL-ETHANAMINIUM DE DERIVES D'ACIDES 5$g(b)-CHOLANE-24

(30) Priorität: 12.09.1988 DE 3830932
(43) Veröffentlichungstag der Anmeldung: 19.09.1990
(73) Patentinhaber: PHARMORGANA GmbH, 83064 Raubling/Rosenheim (DE)
(72) Erfinder: MESSERSCHMITT, Thilo, D-8098 Pfaffing (DE); MÄKE, Siegfried, D-8201 Raubling (DE); AIGNER, Arno, D-8200 Rosenheim (DE); VOGT, Hubert, D-8201 Raubling (DE); BAUER, Adolf, D-8201 Raubling (DE)
(74) Vertreter: Reinhard, Horst, Dr.
(86) Internationale Anmeldenummer: EP8901023
(87) Internationale Veröffentlichungsnummer: WO9002748

(56) Entgegenhaltungen:
- DE-C- 593 258
- FR-A- 2 074 736
- US-A- 2 589 840
- MERCK INDEX 9.Edit. (1976) Seiten 284, 285
- A.AIGNER, A.BAUER Medizinische Monatsschrift für Pharmazeuten 11 (369-375) 1988

## Beschreibung

Beansprucht sind die 2-Hydroxy-N,N,N-trimethyl-ethanaminium salze von 5β-Cholan-24-säure-Derivaten der Formeln I bis III : 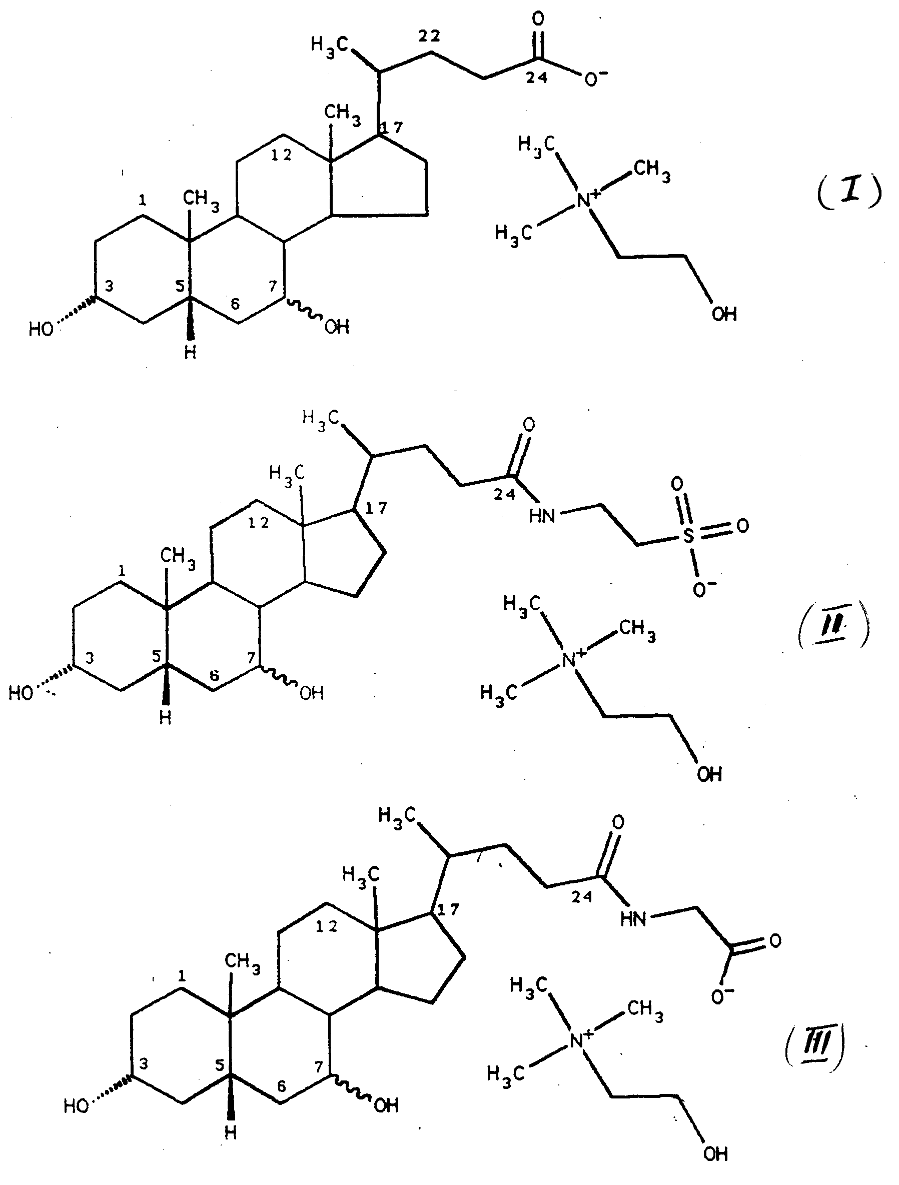
wobei sich die OH-Gruppe der 7-Position jeweils in α- oder β-Stellung befinden kann.

2-Hydroxy-N,N,N-trimethylethanaminiumsalze (=Cholinsalze) der allgemeinen Formeln I bis III sind pharmakologisch wirksame Substanzen, die beispielsweise als Wirkstoffe von Cholagoga oder Gallenwegstherapeutika verwendet werden können.

Aus dem Stand der Technik bekannt sind u.a. die Cholinsalze der Cholsäure, Desoxycholsäure sowie der Dehydrocholsäure, die eine abführende bzw. choleretische Wirkung Laben (DE-C-593258, FR-A-2074736).

Die Herstellung der 2-Hydroxy-N,N,N-trimethylethanaminiumsalze (=Cholinsalze) der allgemeinen Formeln I bis III erfolgt unter Bedingungen, wie sie dem Fachmann wohlbekannt sind.

So kann man beispielsweise die freien Carbonsären in einem polaren inerten Lösungsmittel mit Cholin umsetzen. Geeignete Lösungsmittel sind beispielsweise niedere Alkohole (Methanol, Ethanol, Propanol, Isopropanol etc.) oder niedere Ketone (Aceton, Methylethylketon ). Andererseits sind auch dipolare aprotische Lösungsmittel (Dimethylformamid, Hexamethylphosphorsäuretriamid etc.) geeignet. Diese erbringen aber in der Regel keine Vorteile. Verwendet man als Lösungsmittel niedere Alkohole und etwa 0,2 bis 1 mol Saure pro mol Cholin, so kann man die Salze in einfacher Weise isolieren, indem man das Reaktionsgemisch eindampft und/oder die Reaktionsprodukte durch Zugabe unpolarer Losungsmittel, wie zum Beispiel Aceton ausfällt.

Andererseits kann man auch die Alkalimetallsalze der Cholan-24-säure-Derivate (vorzugsweise die Natrium- oder Kaliumsalze) mit Cholinsalzen anorganischer Säuren (vorzugsweise Cholinchlorid oder Cholinsulfat) umsetzen. Diese Umsetzung wird ebenfalls in inerten polaren Lösungsmitteln durchgeführt. Führt man die Reaktion in einem der oben genannten niederen Alkohole unter Verwendung etwa äquimolarer Mengen beider Reaktionspartner durch, so kann man das Reaktionsprodukt nach Abfiltrieren des Alkalimetallsalzes der anorganischen Säure in einfacher Weise isolieren, indem man das Filtrat einengt und/oder durch Zugabe unpolarer Lösungsmittel, wie zum Beispiel Aceton ausfällt.

Diese Reaktionsvariante läßt sich selbstverständlich auch in der Weise durchführen, daß man die freien Säuren mit einem Cholinsalz einer anorganischen Säure in einem inerten Lösungsmittel, beispielsweise in Gegenwart von Alkalimetallhydrogenkarbonaten (Natriumhydrogenkarbonat oder Kaliumhydrogenkarbonat) umsetzt.

Die 2-Hydroxy-N,N,N-trimethylethanaminiumsalze der allgemeinen Formeln I bis III besitzen die gleiche pharmakologische Wirkung, wie die 5β-Cholan-24-säure-Derivate. Darüberhinaus zeigen sie eine bakterizide oder virizide Wirksamkeit.

Die erfindungsgemäßen Verbindungen zeichnen sich aber unter anderen durch eine gute Wasserlöslichkeit, eine gute Stabilität, eine relativ geringe Basizität, eine relativ gute Resorbierbarkeit aus.

Zur Herstellung von Arzneimittelspezialitäten werden die erfindungsgemäßen Verbindungen gegebenenfalls mit den üblichen Zusätzen und Geschmackskorrigentien zu Tabletten, Dragees und Kapseln verarbeitet, die normalerweise 50 bis 500 mg Wirkstoff pro Dosiseinheit enthalten.

Wenn die Arzneimittelspezialität zur medikamentösen Auflösung von Cholesterin-Gallensteinen zur Prophylaxe der Gallensteinbildung bzw. als bakterizide oder virizide Mittel dienen soll, werden dem Patienten im allgemeinen 5 bis 20 mg Wirkstoff pro kg Körpergewicht und Tag verabreicht.

Die hohe Wasserlöslichkeit der erfindungsgemäßen Substanzen erlaubt darüber hinaus auch die Herstellung wässriger Lösungen, die beispielsweise zur Infusion oder Injektion geeignet sind, welche man beispielsweise zur Behandlung von primärer biliärer Zirrhose und primärer selerosierender Cholangitis sowie auch zur Behandlung von organbedingten akuten Störungen des Gallenflusses und der Darmresorption vorteilhaft anwenden kann. oder welche zur Spülung des Gallengangs oder der Gallenblase mittels percutan transhepatischer Drainage geeignet sind.

Darüberhinaus eignen sich die erfindungsgemäßen Substanzen auch zur Verarbeitung in Wasser/Öl und/oder Öl/Wasser Emulsionen. Die so gebildeten Lotionen, Cremes oder Salben eignen sich als dermatologische Präparate beispielsweise zur Wundbehandlung oder als Kosmetika. Die Herstellung dieser Lotionen, Cremes oder Salben erfolgt nach Methoden, wie sie dem Fachmann wohl bekannt sind (Dr. J. Stephan Jellinek "Kosmetologie" 2. Auflage, 1967, Dr. Alfred Hüthig Verlag, Heidelberg (DE) und Europäische Patentschrift 0065 929).

Die erfindungsgemäßen Substanzen eignen sich ferner als galenische Hilfsmittel für pharmazeutische Zubereitungen von Wirkstoffen um deren transdermale Resorption zu verbessern.

Die nachfolgenden Ausführungsbeispiele dienen zur Erläuterung des erfindungsgemäßen Verfahrens.

### Beispiel 1

21,6 g Natriumsalz der 3α, 7β-Dihydroxy-5β-cholan-24-säure (= 0,052 mol) werden unter Erwärmen in 250 ml Ethanol gelöst, mit einer Lösung von 7,0 g Cholinchlorid in 50 ml Ethanol versetzt und 2 Stunden lang aufbewahrt. Dann läßt man die Reaktionsmischung erkalten, filtriert das ausgefallene Kochsalz ab und engt das Filtrat weitgehend ein und tropft den noch fließfähigen Rückstand in 0.5 l Aceton ein. Man rührt die Mischung noch 2 Stunden lang, saugt das Produkt ab und trocknet es im Vakuum. Man erhält so 17,9 g 2-Hydroxy-N,N,N-trimethylethanaminiumsalz der 3α,7β-Dihydroxy-5β-cholan-24-säure mit einem Schmelzpunkt von 185 bis 190° C.

### Beispiel 2

6,05 g Cholin (= 0,05 mol) werden in 50 ml Ethanol gelöst, mit 19,6 g 3α, 7β-Dihydroxy-5β-cholan-24-säure (= 0,05 mol) versetzt und nach erfolgter Lösung zur Trockne eingeengt. Der Rückstand wird in Aceton aufgenommen, das abgeschiedene Kristallisat aubgesaugt und getrocknet. Man erhält 21,6 g 2-Hydroxy-N,N,N-trimethylethanaminiumsalz der 3α,7β-Dihydroxy-5β-cholan-24-saure mit einem Schmelzpunkt von 186 bis 190° C.

### Beispiel 3

6,05 g Cholin (0,05 mol) werden in 50 ml Ethanol gelöst, mit 19,6 g 3α,7α-Dihydroxy 5β-cholan-24-säure (= 0,05 mol) versetzt und nach erfolgter Losung in 200 ml Aceton eingetropft. Man rührt noch eine Stunde lang, saugt das abgeschiedene Kristallisat ab und trocknet es im Vakuum. Man erhält so 20,0 g 2-Hydroxy-N,N,N-trimethylethanaminiumsalz der 3α, 7α-Dihydroxy-5β-cholan-24-säure mit einem Schmelzpunkt von 70 bis 75° C.

### Beispiel 4

4,6 g N-(3α,7α-Dihydroxy-24-oxo-5β-cholan-24-yl)-glycin (= 0,00832 mol) werden in 10 ml Ethanol gelöst und mit einer Lösung von 1,0 g Cholin-Base (= 0,00832 mol) in 10 ml Ethanol versetzt. Dann tropft man die Mischung in 200 ml Aceton ein, rührt noch einige Zeit. saugt die Kristalle ab und trocknet sie im Vakuum.

Man erhält so 3,6 g 2-Hydroxy-N,N,N-trimethylethanaminiumsalz des N-(3α, 7α-Dihydroxy-24-oxo-5β-cholan-24-yl)-glycins mit einem Schmelzpunkt von 80 bis 83° C.

### Beispiel 5

8,04 g 2-[(3α,7β-Dihydroxy-24-oxo-5β-cholan-24-yl)-amino]-ethansulfonsäure (= 0,0133 mol) werden in 30 ml Ethanol gelöst und mit einer Lösung von 1,61 g Cholin-Base (= 0,0133 mol) in 16 ml Ethanol versetzt. Dann tropft man die Mischung in 200 ml Aceton ein, rührt noch einige Zeit, saugt die Kristalle ab und trocknet sie im Vakuum.

Man erhält so 6,9 g 2-Hydroxy-N,N,N-trimethylethanaminiumsalz der 2-[(3α,7β-Dihydroxy-24-oxo-5β-cholan-24-yl)-amino]-ethansulfonsäure mit einem Schmelzpunkt von 80 bis 85° C.

## Patentansprüche

1. 2-Hydroxy-N,N,N-trimethylethanaminiumsalze von 3α-7-Dihydroxy-5β-cholan-24-säuren der Formel (I) 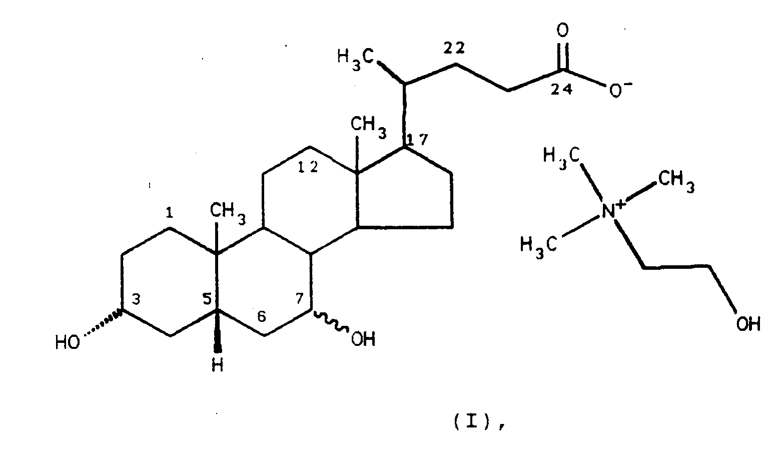 wobei sich die OH-Gruppe der 7-Position in α- oder β-Stellung befinden kann.

2. 2-Hydroxy-N,N,N-trimethylethanaminiumsalze von 2-[(3α-7-Dihydroxy-24-oxo-5β-cholan-24-yl)amino]ethansulfonsäure der Formel (II) 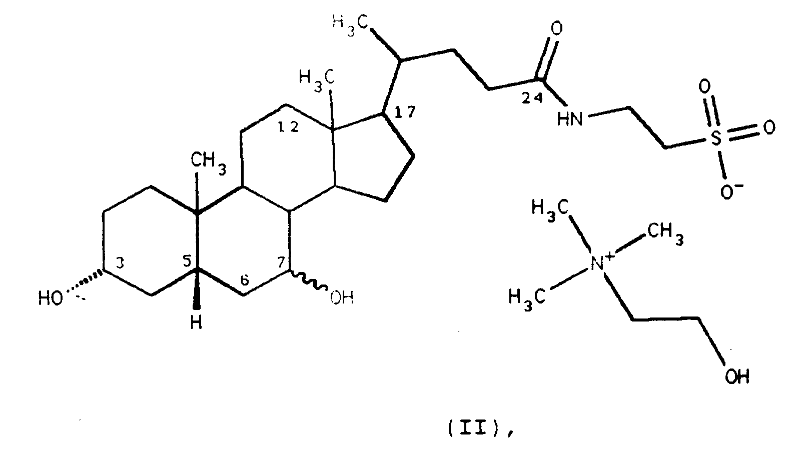 wobei sich die OH-Gruppe der 7-Position in α- oder β-Stellung befinden kann.

3. 2-Hydroxy-N,N,N-trimethylethanaminiumsalze von N-(3α-7-Dihydroxy-24oxo-5β-cholan-24-yl)glycin der Formel (III) 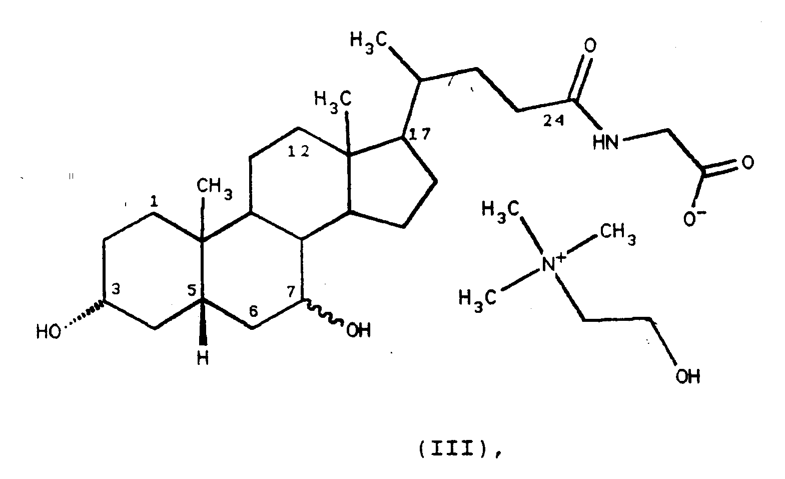 wobei sich die OH-Gruppe der 7-Position in α- oder β-Stellung befinden kann.

## Claims

1. 2-hydroxy-N,N,N-trimethylethanaminium salts of 3α-7-dihydroxy-5β-cholanic-24 acids of formula (I) 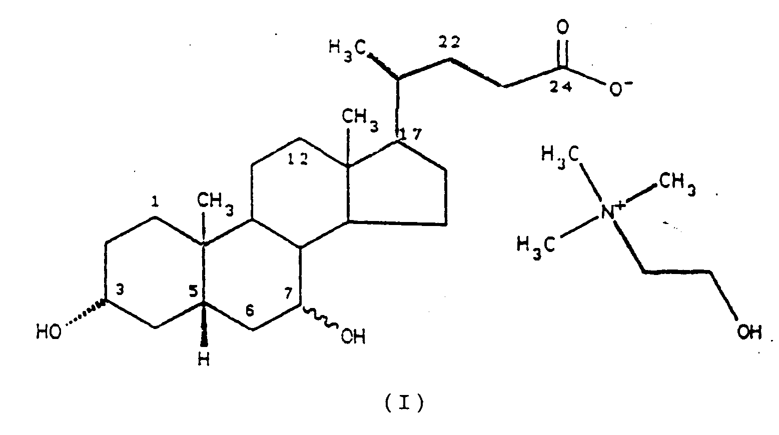 wherein the OH group of position 7 can be in α or β position.

2. 2-hydroxy-N,N,N-trimethylethanaminium salts of 2-[(3α-7-dihydroxy-24 oxo-5β-cholane-24-yl)amino]ethane sulfonic acids of formula (II) 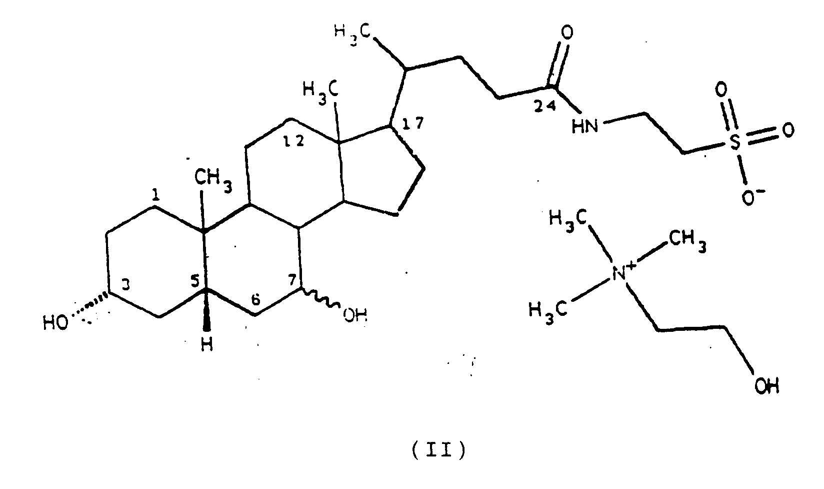 wherein the OH group of position 7 can be in α or β position.

3. 2-hydroxy-N,N,N-trimethylethanaminium salts of N-(3α-7-dihydroxy-24-oxo-5β-cholane-24-yl)glycine of formula (III) 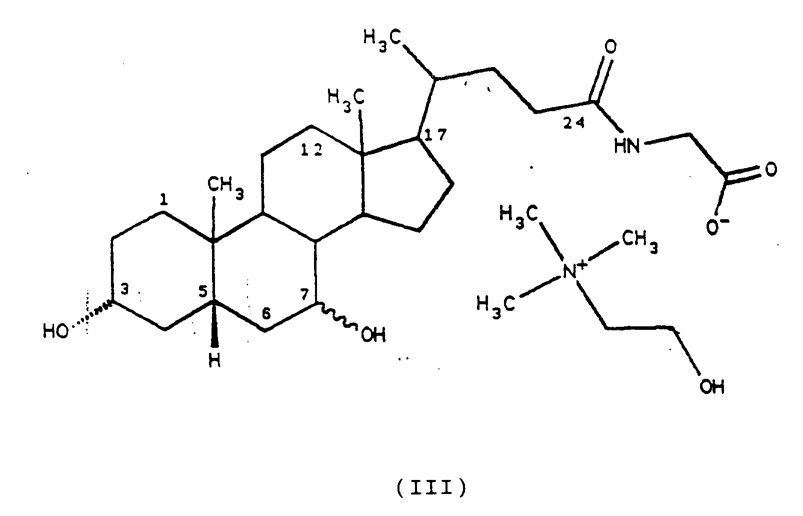 wherein the OH group of position 7 can be in α or β position.

## Revendications

1. Sels 2-hydroxy-N,N,N-triméthyléthanammonium d'acides 3α-7-dihydroxy-5β-cholane-24-carboxyliques de formule (I) 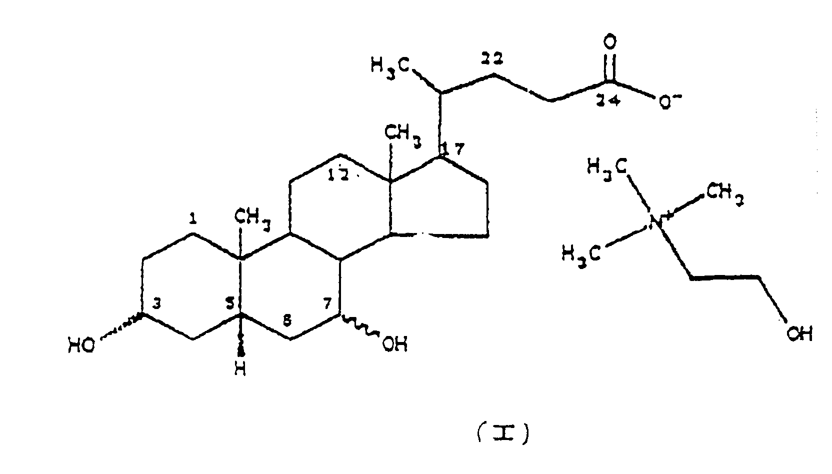 où le groupe OH en position 7 peut être placé en position α ou β.

2. Sels 2-hydroxy-N,N,N-triméthyléthanammonium d'acides 2-[(3α-7-dihydroxy-24-oxo-5β-cholane-24-yl) amino] éthanesufoniques de formule (II). 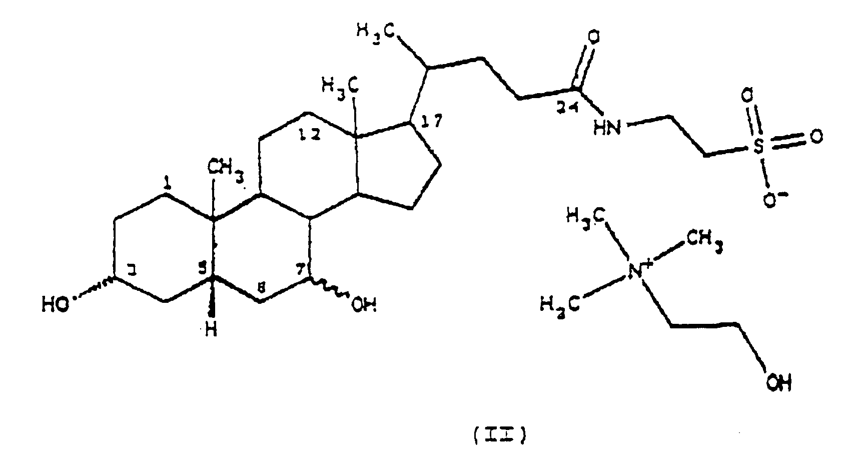 où le groupe OH en position 7 peut être placé en position α ou β.

3. Sels 2-hydroxy-N,N,N-trimethyléthanammonium d'acides N-(3α-7-dihydroxy-24-oxo-5β-cholane, 24-yl) glyciniques de formule (III). 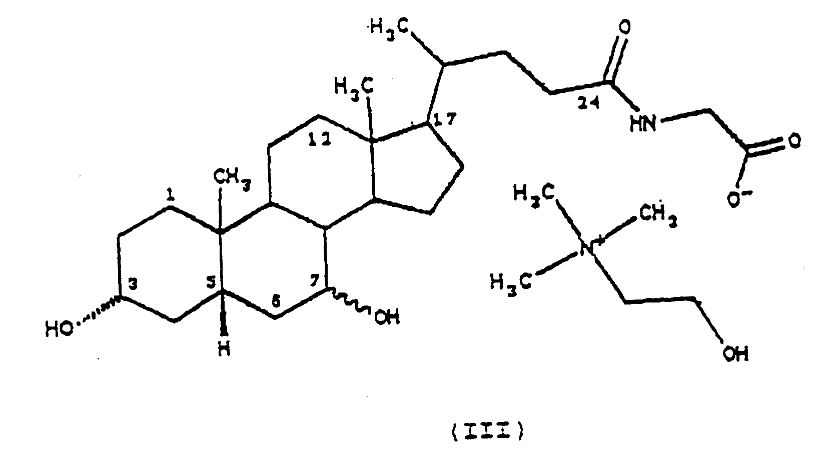 où le groupe OH en position 7 peut être placé en position α ou β.
